(19) Europäisches Patentamt
European Patent Office
Office européen des brevets

(11) **EP 1 101 114 B1**

(12) **EUROPEAN PATENT SPECIFICATION**

(45) Date of publication and mention
of the grant of the patent:
**12.10.2005 Bulletin 2005/41**

(21) Application number: **99936801.2**

(22) Date of filing: **29.07.1999**

(51) Int Cl.[7]: **G01N 33/53**

(86) International application number:
**PCT/GB1999/002478**

(87) International publication number:
**WO 2000/007024 (10.02.2000 Gazette 2000/06)**

(54) **METHOD OF PROTEIN ANALYSIS**

VERFAHREN ZUR EIWEISSANALYSE

METHODE D'ANALYSE DE PROTEINES

(84) Designated Contracting States:
**AT BE CH CY DE DK ES FI FR GB GR IE IT LI LU MC NL PT SE**

(30) Priority: **29.07.1998 GB 9816514**

(43) Date of publication of application:
**23.05.2001 Bulletin 2001/21**

(73) Proprietor: **SMITHKLINE BEECHAM PLC Middlesex TW8 9GS (GB)**

(72) Inventors:
• **WILDSMITH, Sophie, Elizabeth, Victoria Welwyn, Hertfordshire AL6 9AR (GB)**
• **YALLOP, Rhiannon Welwyn, Hertfordshire AL6 9AR (GB)**
• **BUGELSKI, Peter, John Welwyn, Hertfordshire AL6 9AR (GB)**
• **MORGAN, David, Gwyn King of Prussia, PA 19406 (US)**

(74) Representative: **Blakey, Alison Jane et al GlaxoSmithKline Corporate Intellectual Property Two New Horizons Court Brentford, Middlesex TW8 9EP (GB)**

(56) References cited:
EP-A- 0 376 851          WO-A-98/29744
WO-A-99/39210

• LEUNG, SAU-MEI (1) ET AL: "A new and rapid method for phenotype screening using SELDI Protein Chip arrays demonstrated on serum from knockout and wildtype mice." MOLECULAR BIOLOGY OF THE CELL, (NOV., 1998) VOL. 9, NO. SUPPL., PP. 351A. MEETING INFO.: 38TH ANNUAL MEETING OF THE AMERICAN SOCIETY FOR CELL BIOLOGY SAN FRANCISCO, CALIFORNIA, USA DECEMBER 12-16, 1998 AMERICAN SOCIETY FOR CELL BIOLOGY. , XP000874716
• BLACKSTOCK W P ET AL: "Proteomics: quantitative and physical mapping of cellular proteins" TRENDS IN BIOTECHNOLOGY,NL,ELSEVIER, AMSTERDAM, vol. 17, no. 3, page 121-127 XP004157732 ISSN: 0167-7799
• ANDERSON N L AND ANDERSON N G: "Proteome and proteomics: New technologies, new concepts, and new words" ELECTROPHORESIS,DE,WEINHEIM, vol. 19, no. 11, - 1998 pages 1853-1861, XP002115233 ISSN: 0173-0835
• HUMPHERY-SMITH ET AL: "Proteome research:Complementarity and limitations with respect to the RNA and DNA worlds" ELECTROPHORESIS, vol. 18, no. 8, - May 1997 (1997-05) pages 1217-1242, XP000870390
• S R PENNINGTON ET AL: "Proteome analysis: from protein characterization to biological function" TRENDS IN CELL BIOLOGY,XX,ELSEVIER SCIENCE LTD, vol. 7, no. 7, April 1997 (1997-04), page 168-173 XP002103064 ISSN: 0962-8924

- **DAVIES J ET AL: "Affinity improvement of single antibody VH domains: residues in all three hypervariable regions affect antigen binding" IMMUNOTECHNOLOGY,NL,ELSEVIER SCIENCE PUBLISHERS BV, vol. 2, no. 3, - 1996 page 169-179 XP004070292 ISSN: 1380-2933**

- **DAVIES J ET AL: ""Camelising" human VH domains for use as small recognition units. [Abst. 207]" IMMUNOTECHNOLOGY,NL,ELSEVIER SCIENCE PUBLISHERS BV, vol. 2, no. 4, - 1996 page 285 XP004063194 ISSN: 1380-2933**

**Description**

The invention relates to a method that makes use of rapid analysis of the protein or peptide composition of a mixture, in particular analysis of an extract derived from tissues or cells in order to measure the differential expression or abundance of proteins. The invention can be used in the analysis of the temporal and spatial changes in protein expression in response to drug treatments when investigating drug induced toxicity.

Proteins and peptides play a central role in many cellular processes in all cellular biosystems. Examples of such functions include structural and catalytic roles. The 'proteome', that is the protein expression pattern in a given tissue or cell type, is a notional reflection of the dynamic nature of these cellular systems. Genes are constantly being induced or switched off to modulate the levels of different proteins within the cell and these proteins are sometimes modified or secreted from the cell to act as signals to other cells. These signals in turn alter the gene expression pattern of the receiving cell. These dynamic systems, despite being very tightly controlled are, however, inherently susceptible to disturbance. Bacterial or viral infection, for example, results in a change in this equilibrium as the system attempts to repel the invading micro-organisms and restore the system to normal. The delicate balance of cellular processes can also be disturbed as a result of genetic abnormalities. Similarly, environmental insults such as physiological trauma and exposure to chemicals can alter the proteome of affected tissues and cells. Such disturbance of the qualitative, quantitative, temporal and spatial distribution of proteins and peptides are frequently associated with serious cellular dysfunction and can lead to tissue damage and in some cases death,

The disturbances of cellular processes, as reflected in the proteome of the cell or tissue are of particular concern in the pharmaceutical industry in the safety assessment of new drugs. For example ingestion of a novel chemical compound is an environmental insult. Many new drugs are identified by screening large collections of chemical compounds for a compound that demonstrates an ability to block an interaction between two components of a cellular process. In a patient suffering from a condition where this interaction is unwanted or is excessive, due to abnormally elevated levels of one of the components, reducing the levels of this interaction by treating the patient with a blocking drug will restore the system to equilibrium, Therefore, by their very nature, many drugs will affect the balance of proteins in the cell or tissue. However many drugs, in addition to effecting the desired activity upon a cellular process, also have unwanted effects on other cellular processes (so called 'side effects'), and this will be reflected in the change in the proteome. In determining the safety novel compounds it is necessary to examine and compare the proteomes from treated and untreated animals, tissues or cells, and identify any changes that have taken place as a result of administering the new drug. It would also be an advantage to be able to identify the particular proteins whose expression is affected by the treatment.

Detection of proteomic changes is not a trivial task despite the existence of several protein anaylsis techniques. Existing techniques fall into the following catagories:

(1) Quantitative, non-specific, total proteins: Lowry, Bradford, Coomassie, BCA assays, for quantitation of proteins in solution.
(2) Qualitative (can be made quantitative): staining of gels with silver stain, Coomassie stain, radioactivity and fluorescent staining.
(3) Quantitative and specific: e.g.immunological methods such as detection of antigens by ELISA, immunoprecipitation and Western blots.
(4) Qualitative and quantitative (non-specific): high pressure liquid chromatography (hplc), fast protein chromatography (fpc), capillary zone electrophoresis (cze).
(5) Qualitative, high accuracy; mass spectrometric methods e.g. (MALDITOF).

One technique which is currently used is 2-Dimensional (2-D) gel electrophoresis. However this technique is laborious, requires skilled staff, is not easily reproducable and the analysis of data is complex. Proteins, which are extracted from a tissue or cells, are separated in a gel in one dimension based upon their isoelectric point. They are further separated by electrophoresis, in a second dimension, on the basis of their molecular weight. The gels are stained, using either conventional protein stains, such as Coomassie blue or silver stain, or by more sensitive fluorescent stains. The result is a slab of gel, usually approximately 20cm x 30cm with a multitude of "spots". This is scanned using the appropriate wavelength of light (see for example Anderson et al, Tox. Path, 1996, 24, 72-76). Complex software is required to locate spots, differentiate between discrete spots and artifacts and to identify the proteins of interest. Gels from different samples can be compared, e.g. from treated and untreated tissues, but problems can arise in loacting indentical spots due to distortion of the gel and other reproducibility problems. Not every protein in a sample will be identified using this technique - only a certain pH range can be examined at any one time there will be no limit to the size range which can be looked at on one gel. Moreover some proteins do not stain with certain dyes.

In EP0167335A2, a method of detecting a binding reaction between an antigen and an antibody is disclosed. The antibody is immobilized onto a two dimensional surface so as to form a spatial array. The binding reaction is detected using optical means.

None of the currently available methods enable a high throughput, qualitative and quantitative analysis of proteomic change. The present invention makes use of method for analyzing the protein or peptide content of a mixture which enables a high throughput, qualitative and quantitative analysis of proteomic change.

According to the invention there is provided a method of predicting the toxic effect of a selected test compound on a mammalian subject, said method comprising the steps of

(a) providing a plurality of identical spatial arrays of protein recognition units;

(b) exposing a spatial array of step (a) to a protein or peptide containg mixture extracted and isolated from a target cell, tissue or biological organism that has been exposed to a known torxicant and detecting the binding of proteins or peptides to the protein recognition units;

(c) exposing a spatial array of step (a) to a protein or peptide containg mixture extracted and isolated from a targel cell, tissue or biological organism that has not been exposed to the known tocicant of step (b) and detecting the binding of proteins or peptides to the protein recognition units;

(d) comparing the binding pattern of step (b) and step (e) to identify any difference in binding between the target exposed to the known toxicant and the non-exposed target, said difference being indicative of an effect on protein expression in the target exposed to said known toxicant;

(e) repeating steps (b), and (d) optionally step (c) with another known and structurally distinct toxicant having a common toxic effect on mammalian subjects as the known toxicant of step (b) so as to generate a "fingerprint" binding pattern characteristics of said common toxic effect;

(f) repeating steps (b) and (d), optionally step (c) with said selected test compound to generate a test binding pattern wherein substantial identity between the fingerprint of step (e) and the test binding pattern indicates that said test compound shares said common toxic effect.

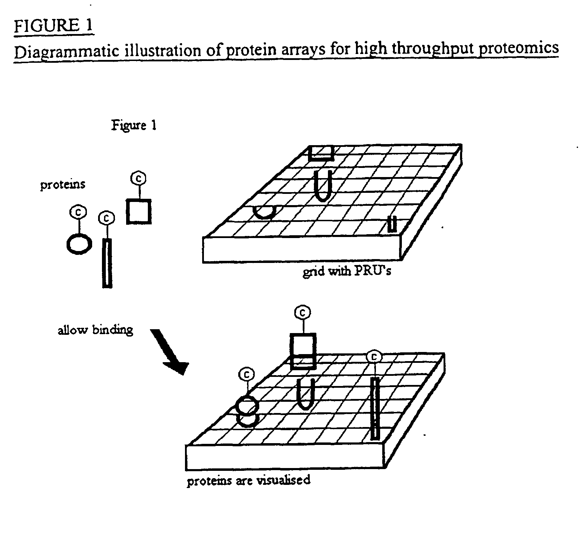

Figure 1 shows a diagrammatic representation of the method of the invention. Protein recognition units (PRU's) are immobilized or synthesized on a grid system. Four PRU's are shown, but the number of PRU's may vary depending on the application e.g. low density (<100 PRU/cm$^2$) or high density (>100 PRU/cm$^2$). Proteins are recognized by, and bind to, the PRU's. The proteins are pre- or post-labelled so that a signal from a given grid co-ordinate indicates that the protein which corresponds to that PRU is present.

The spatial array ofPRUs preferably comprises a plurality of different PRUs. The PRUs are preferably immobilized on a solid or semi-solid support. The PRUs are preferably arranged in a specific, coordinated pattern, e.g. in a grid format, such that the location of each PRU is known. The PRUs may be minimal recognition units (MRUs), antibodies or antibody fragments, high affinity peptides or complementarity determining regions (CDRs). The PRUs may be of known or unknown sequence content i.e. the identity of the PRU or bound protein can be determined retrospectively, if desired, by releasing the PRU from the solid surface, separating the bound protein and sequencing both.

The PRUs are preferably CDRs. In native IgG antibodies the variable heavy and light chains each contribute three regions called CDR's which are responsible for binding antigen. Each region is typically 7-20 amino acids long, e.g. 15-17 amino acids, and its sequence defines the specificity and affinity of that CDR for the antigen. There is increasing evidence that these regions of CDR-peptides are capable of autonomous specific binding to antigens, see for example Steinbergs J, Kilchewska K, Lazdina U, Dishlers A, Ose V, Sallberg M, Tsimanis A, *Hum. Antibod. Hybridomas,* 1996, 7, 3; William WV, Kieber-emmons T, VonFeldt J, Greene MI, Weiner DB, *J. Biol. Chem.*, 1991, **266**, 5182; Saragovi HU, Fitzpatrick D, Raktabutr A, Nakanishi H, Kahn M, Greene M, *Science,* 1991, **253**, 792, Welling WG, van Gorkum J, Damhof RA, Drijhout JW, Bloemhoff W, Welling-Wester S, *J. Chromatogr.,* 1991, **548,** 235; and Levi M, Sallberg M, Ruden U, Herlyn D, Maruyama H, Wigzell H, Marks J, Wahren B, *Proc. Natl. Acad. Sci. USA.,* 1993, **90**, 4373; and hence they have been proposed as new generation antibody fragments with reduced immunogenicity and as anti-viral molecules, see Sivolapenko GB, Douli V, Pectasides D, Skarlos D, Sirmalis G, Hussain R, Cook J, Courtney-Luck NS, Merkouri E, Konstantinides K, Epenetos AA, *Lancet* 1995, **346,** 1662; and Rossenu S, Dewitte D, Vandekerckhove J, Ampe C, *Journal of Protein Chemistry*, 1997, **16**, No.5.

Antibody fragments may, for example, be generated using phage display technologies. CDRs can be produced by recombinant means or can be chemically synthesized. CDRs can be chemically synthesized according to known CDR sequences using either standard protein synthesis or using a combinatorial synthesis approach.

CDR sequences may be determined by analysis of various numbering systems such as the Kabat, Chothia, AbM and Contact numbering schemes:

The Kabat definition is based on sequence variability and is the most commonly used. The Chothia definition is based on the location of the structural loop regions.

The AbM definition is a compromise between the two used by Oxford Molecular's *AbM* antibody modeling software. The Contact definition is specified by University College London and is based on an analysis of the available complex crystal structures. Mean contact data is produced by an analysis of the contacts between antibody and antigen in the complex structures available in the Protein Databank (MacCallum, R. M., Martin, A. C. R. and Thornton, J. T., *J. Mol. Biol.*, 262, 732-745). Briefly, the number of contacts made at each position, defining contact as burial by > 1 square Angstrom change in solvent accessibility. These data give a simple measure of how likely a residue is to be involved in antigen contact.

H3 and L3 make the most contacts with their antigen because they are central in the combining site. H1, L1 and H2 make fairly similar numbers of contacts being rather more peripheral with L2 making the fewest contacts overall. Topographically it is in an equivalent position to H2 (the light and heavy chains have a rotational axis of pseudo-symmetry), but it is much shorter so has less chance of reaching the antigen. L3 makes a lot of contacts but they tend to be generally "sticky" rather than specific. The following table lists the positions of the CDRs according to each numbering system. "L" refers to "light chain" and "H" to "Heavy chain" and the numbers correspond to amino acid sequences.

Table of CDR Definitions

| Loop | Kabat | AbM | Chothia | Contact |
|------|-------|-----|---------|---------|
| L1 | L24 -- L34 | L24 -- L34 | L24 -- L34 | L30 -- L36 |
| L2 | L50 -- L56 | L50 -- L56 | L50 -- L56 | L46 -- L55 |
| L3 | L89 -- L97 | L89 -- L97 | L89 -- L97 | L89 - L96 |
| H1 | H31 -- H35 | H26 -- H35 | H26 -- H32/34 | H30 -- H35 |
| H2 | H50 -- H65 | H50 -- H58 | H52 -- H56 | H47 - H58 |
| H3 | H95 -- H102 | H95 -- H102 | H95 -- H102 | H93 -- H101 |

CDR sequences can be found by using one of, or a combination of the above mentioned methods. A few amino acids either side of the CDR sequence can also be used as these can also enhance binding. The methods may be applied to antibody light and heavy chain protein sequences from any of protein search sites e.g. Entrez, Swiss-Prot. If the protein sequence for a desired antibody is not available the nucleotide sequence can be translated into peptide sequence by using a package such as Lasergene. (Dnastar - www.Dnastar.com.)

In operation the spatial array of PRUs is exposed to the protein or peptide containing mixture, e.g. as a tissue or cell extract, under conditions favourable to protein binding. Proteins or peptides in an extract or mixture are subsequently bound to the grid in a specific manner. The binding of proteins or peptides to the PRUs may be effected by visualization of the proteins or peptides e.g. by staining or other methods such as radiolabelling or chemical labeling, either before or after the binding step. Biotinylation, fluorescence, chemiluminescence or other labeling techniques may also be used. Analysis of the resultant binding pattern may be undertaken using an appropriate detection system such as optical or laser scanning technology and a comparison of results between, for example, drug treated and untreated control tissues can be undertaken using suitable computer software.

The protein or peptide containing mixture that is analyzed according to the method of the invention is preferably a cell extract isolated from a biological organism, e.g. from mammalian tissue. The cell extract is more preferably from a mammal treated with a chemical compound of therapeutic potential.

The method of the invention provides a fast, reproducible and robust alternative to the methods currently used for determining the protein expression pattern of tissues or cell types, i.e. proteomics, applied to toxicology. The method is related to methods that may be used for analyzing the spatial and temporal changes in the expression of proteins in mammalian tissues and cell types when exposed to environmental stimuli such as pharmaceutical reagents, as well as other environmental stimuli or factors. The method is related to methods that allow for the comparison of targets treated with therapeutic drugs and untreated controls to provide a quantitative and/or semi-qualitative analysis of the proteome of the treated and untreated (control) target systems e.g. cells, tissues or biological organisms. The method is also related to methods that may be adapted for use in, for example, drug manufacturing, purity analysis (e.g. in food and agriculture) and for diagnostics, e.g. for detecting proteins in urine or blood.

Thus the invention is also related to a method of determining the effect of an environmental factor on protein expression in a target cell, tissue or biological organism, said method comprising the steps of:

(a) providing a plurality of identical spatial arrays of protein recognition units;

(b) exposing a spatial array of protein recognition units to a protein or peptide containing mixture extracted and isolated from a target that has been exposed to an environmental factor for a sufficient tissue to affect protein expression in said target, and detecting the binding of proteins or peptides to the protein recognition units;

(c) exposing a spatial array of protein recognition units to a control protein or peptide containing mixture extracted and isolated from a target that has not been exposed to said environmental factor and detecting the binding of proteins or peptides to the protein recognition units; and

(d) comparing the first and second binding patterns detected in steps (b) and (c) to identify any change in said first pattern from the control pattern, indicative of an effect on protein expression in the target exposed to said environmental factor.

The terms environmental factor as used herein describes a wide variety of physical, chemical or biological factors which may cause changes in protein expression in a target, e.g.cell, tissue or biological organism, when exposed to that factor. For example, physical environmental stimuli can include, without limitation, diet, an increase or decrease in temperature, an increase or decrease in exposure to ionizing or ultraviolet radiation, and the like. Biological/chemical stimuli can include, without limitation, administering a transgene to the target, or eliminating a gene from the target; administering an exogenous synthetic compound or exogenous agent or an endogenous compound, agent or analog thereof to the target.

As an example, an exogenous synthetic compound can be e.g. a pharmaceutical compound, a toxic compound, a protein, a peptide, or a chemical composition. An exogenous agent can include natural pathogens, such as microbial agents, which can alter protein expression. Examples of pathogens include bacteria, viruses, and lower eukaryotic cells such as fungi, yeast, molds and simple multicellular organisms, which are capable of infecting a target and replicating its nucleic acid sequences e.g. in the cells or tissue of that target. Such a pathogen is generally associated with a disease condition in the infected mammal.

An endogenous compound is a compound which occurs naturally in the body. Examples include hormones, enzymes, receptors, ligands, and the like. An analogue is an endogenous compound which is preferably produced by recombinant techniques and which differs from said naturally occurring endogenous compound in some way.

The invention and methods related to the invention will now be illustrated by way of the following examples.

Example 1: Protein Array with CDR's as Protein Recognition Units and fluorescent dye detection

1.1 Preparation of spatial arrays

CDR's are synthesized according to known peptide synthesis techniques e.g. Sällberg *M et al. Immunol. Lett.*, 1991, **30**, 59. Peptides can be analyzed and purified, for example by reverse phase HPLC as described by Steinbergs *et al. Hum. Antibod. Hybridomas,* 1996, **7(3),** 106-112. CDRs can either be synthesized according to known sequences from literature or experimentation or they can be synthesized as unknowns. This can be either a random synthesis or a combinatorial synthesis. For a CDR of 16 amino acids, the theoretical number of all possible permutations exceeds what would be manageable in practice ($1.8 \times 10^9$ possibilities). This number can be reduced by incorporating knowledge of CDR structures, common motifs and probabilities of sequences into the decision making process. Examples of different approaches are a) removing sequences featuring the same amino acid more than a certain number of times (or in succession), or b) limiting amino acid usage to that similar to the natural or known occurrence in CDR's. An alternative to this informed or "intelligent" combinatorial approach is a pre-screened combinatorial approach. In this case, known proteins of interest are used to pull out corresponding CDR's from a random synthesis and therefore the CDR sequence need not be known.

The CDRs once synthesized can be arrayed by applying them to a support in any format wherein the CDRs are arrayed in a spatial manner. The CDRs are preferably gridded. The support can comprise a gel, plastics, nylon, nitrocellulose, glass, silica, carbon, perfluorocarbons, alumina, ceramics, polymers, cellulose, crosslinked polysaccharides, metals or any other material on which the CDRs can be immobilized. Standard methods for immobilization are well documented (e.g. Mosbach, Methods in Enzmology, 1976, 44; Immobilized biochemicals and affinity chromatography, advances in experimental medicine and biology, Ed R.Dunlap, Plenum Press, N.Y., 1974; 42; and Chemistry of protein conjugation and cross linking. Wong, CRC Press, 1993) and usually exploit the reactive groups on amino acids such as the amine and thiol groups for disulphide linkages, thioether bonds, hindered disulfide bonds and covalent bonds. Other linkage methods include introducing inert amino acids and utilising photoactivation, metal ion linkages and other linkering/anchoring molecules. Preferred immobilization techniques do not disrupt the CDR properties and do not interfere with the protein recognition attributes of the CDRs.

## 1.2 Application of test solution

Once designed and fabricated, the arrays can be used to detect proteins in a mixture. The arrays are flooded with a protein mixture derived from any source, e.g. tissue homogenate, cell free suspension, supematants. The protein mixture can be altered to facilitate binding. For instance, the proteins can be dialysed into a buffer or a solution with a pH and ionic constitution such as to enable binding. Throughout the binding process, the conditions can be altered in order to affect the stringency of binding. A suitable hybridisation buffer is 0.5M phosphate buffered saline, pH7. In this case, stringency can be decreased by lowering the pH of the solution during the binding process. The binding procedure can be allowed to occur over a period of several hours. The protein arrays can then be washed using buffer as above.

## 1.3 Detection of protein binding

The proteins are preconjugated with a fluorescent dye such as Alexa, BODIPY, SYPRO™ Orange or SYPRO™ Red stains from Molecular Probes. Any other fluorescent dyes which bind proteins could be used. The preferred embodiment is a covalent method of attachment such as binding of the primary amine groups (e.g. Alexa, BODIPY dyes). Images of the arrays can be obtained using a laser scanner and CCD acquisition system, for example the STORM™ system from Molecular Dynamics.

An alternative labeling methodology involves radioactive labeling, which may be achieved, for example by adding $S^{35}$ to the growth medium in which proteins are synthesized. In this way nascent proteins will be radiolabelled. When radiolabelled proteins are bound to the array they can be visualized using standard autoradiography techniques.

Images of the arrays can be used to analyze the proteins present and the quantities of proteins present in the samples. If the CDRs are known, then the position of the CDRs on the array is known and the relative concentrations of those proteins can be determined. Controls of known concentration can be used for quantification purposes. If the CDR's are unknowns (generated using a combinatorial approach) then patterns of expression of proteins can be monitored. If certain proteins prove interesting, for example, if the expression level changes with at different stages of a cell cycle, then those proteins can be isolated and identified.

## Example 2: Protein Array with bacteriophage display proteins as Protein Recognition Units

In this method bacteriophage (hereafter "phage") expressing heterologous protein fragments or peptides, including random peptides, on their surfaces are immobilized on a support. The heterologous protein fragments are preferably antibody variable regions. The displayed protein fragments or peptides are thereby enabled to function as PRUs in the method of the invention. The phage-antibody display system preferably allows expression of ScFv or Fabs on the surface of a bacteriophage. Antibody variable region genes are cloned into the genes encoding a coat protein, for example a minor coat protein, of the filamentous bacteriophage thereby creating a fusion protein. The antibody genes are selected from a gene repertoire, for example mRNA extracted from B cells, and expressed in the phage library.

The phage display library thus produced can be used to screen for proteins in mixtures. Binding of proteins to phage displayed antibodies can be achieved using conditions similar to those described by Palmer *et al, Vox Sanguinis*, 1997, **72,** 148-161.

## Example 3: Application of protein arrays to monitor dynamic changes to protein expression induced by pharmaceutical compounds

The invention enables the investigation of changes in protein expression in a tissue or cell-type of interest in response to exposure to a therapeutic compound. This aspect of the invention is facilitated by preparing a series of test animal samples, tissue samples or cell-cultures, introducing the compound and harvesting the cells at selected time points. A cellular extract can then be prepared from the harvested cells using standard cell disruption techniques, in the presence of appropriate protease inhibitors, and the extracted proteins stained with fluorescent stain. The spatial array of PRUs is exposed to the protein extract under conditions allowing proteins in the extract to bind to the PRUs in the array. The array can then be visualized using excitation of the specific wavelength for the fluorophore.

The "pattern" of fluorescence is compared in the treated and untreated cells. Proteins of interest which occur on one array, but not another can be isolated and identified. After some time, the proteins which the PRU's recognize will become known and a database can be constructed which relates the expression of proteins in response to drugs. This database can be used to predict the likely action and toxicity of a compound and possibly it's pharmacology. It will also contribute to an understanding of cascades of events associated with certain drugs.

Example 4: Proteomics Array to differentiate protein expression in response to classic hepatotoxins

Example tissues

The protein expression in response to five classic hepatotoxins is studied using the method of the invention. Antibodies to proteins are used, where the protein expression may differ to the control and a specific pattern of expression can be identified for a given compound. The hepatotoxins used are sodium valproate, erythromycin estolate, methotrexate, acetominophen and coumarin.

Example PRU's

A number of antibodies, fragments or CDR's are immobilized on a solid support. In practice several thousand PRU's could be laid down. In the simplest case, nine antibodies are bound to a sheet of white polystyrene. The antibodies are from a selection of commercially available antibodies such as those to alkaline phosphatase, GST-glutathione transferase, TNF, p53, Bcl-2, hepatocyte growth factor, insulin-like growth factor, folic acid (all available from Sigma). In addition, rat whole serum antibodies can be used for control spots.

Example method of immobilization

The antibodies, in coating buffer (e.g. 0.05 carbonate buffer, pH9.6) are spotted onto the polystyrene sheet (PVDF, nitrocellulose or glass could be used) in triplicate and at 3 different concentrations. They are incubated overnight at 4°C in a humidity chamber to allow binding. The sheet is rinsed and then incubated for one hour with blocking buffer to prevent non-specific absorption (0.05M carbonate buffer, pH9.6, 0.5% casein). The sheet is then washed with wash buffer (sodium phosphate buffer)1 and sample is applied, diluted in phosphate buffered saline to a concentration of 1 mgml-1.

   1. sodium chloride (58.44g), disodium hydrogen orthophosphate (11.53g), sodium dihydrogen orthophosphate dihydrate (2.925g), EDTA (37.22g), Tween 20 (2ml), L-butanol (100ml), distilled water to 10L, pH7.

Example sample preparation

The samples are cell lysates, made from the livers of rats treated with the above hepatotoxins and prepared according to the RiboLyser protocol (Hybaid). The cell lysates prepared in sample buffer containing a mixture of protease inhibitors of 2mM phenylmethylsufonyl flouride, 2ug/ml aprotinin, 2mM sodium orthovanadate, 100mM sodium floride, 10mM sodiumm floride, 10mM sodium pyrophosphate, 10mM sodium molybdate and 2mgm1-1 leupeptin. The lysates are concentrated using a Pierce KwikSpin ultrafilteration unit with a 5kDa cut off before determining the total protein concentration using a Coomassie Plus protein assay (Pierce). The semi-purified protein are conjugated with Texas Red-X (Molecular Probes, product no F- 6162) according to the protocol provided by the manufacturer. Texas Red absorbs at 595nm and emits at 625nm and thus the resulting sheets can be scanned on a Molecular Dynamics Storm system.

Example Results

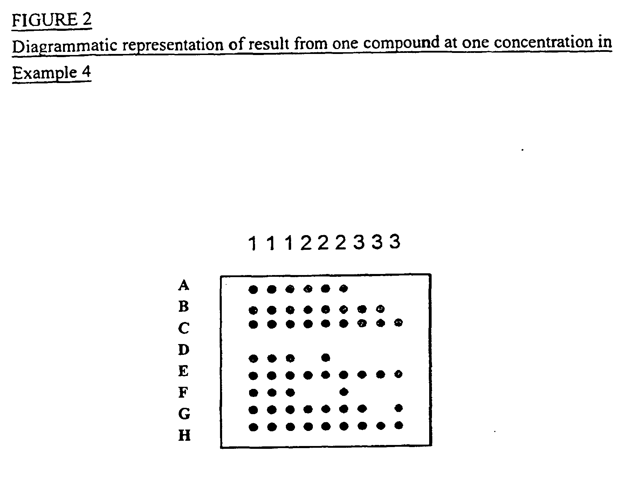

Figure 2 is a diagrammatic example of the resulting scanned sheet where A to H are PRU's spotted in three concentrations from left to right and the spots indicate the protein present in that sample from that tissue.

Example conclusions

These results indicate that no protein D is detected in the sample but there is high expression of protein H. This pattern may enable this compound to be distinguished from the untreated sample and the four other hepatoxicant treated livers. These results can be used as a pattern matching exercise, and changes in expression can be used for developing hypotheses regarding mechanisms of toxicity. Where a large number of PRUs are used, for example using synthetic CDRs, particularly high or low expressing proteins may be identified and their importance recognized for the first time.

Example 5: Proteomics Array to differentiate protein expression in the liver following administratuon of azathioprine compared to positive control

5.1 PRU immobilization/Grid design

Coat Silane coated glass slides (Sigma) with glutaraldehyde 2%(Agar Scientific) and leave for 1 hour. Draw an area on the slide with PAP pen (Sigma) this creates a hydrophobic barrier around the area to be spotted with antibodies or protein recognition units. Spot a solution-containing antibody/ protein recognition unit directly onto the slide in 0.5ul volumes, diluted in coating buffer (0.2M sodium carbonate buffer, pH 9.6 or KPL coating buffer (Kirkegaard and Perry Laboratories)). Leave at 4°C O/N in a humidified container.

PVDF or Nitro-cellulose slide - Oncyte slides (Grace Biolabs) can also be used as a surface these are prepared differently from the above as follows:

Pre-wet PVDF membrane by floating membrane in 100% methanol (ROMIL) and submerging briefly. Transfer membrane to a container of TBS (no Tween 20) & submerge for 2 minutes. To prevent the membrane drying out during spotting, place membrane on top of 2 sheets of blotting paper wet with TBS. Spot a solution containing antigen/ protein recognition unit directly onto moist PVDF membrane in 0.5ul volumes, diluted in coating buffer. Leave for 1 hour RT in humidified container.

Nitro-cellulose slide - Oncyte slides (Grace Biolabs) are prepared by spotting a solution containing antigen/ protein recognition unit directly onto the slide in 0.5ul volumes, diluted in coating buffer. Leave for 1 hour RT.

Custom glass slides with various coatings and wells for the antibody/ protein recognition unit spots cut into the glass may also be used.

5.2 Protein lysate preparation from tissue

Lysates made from 3 treatments of mouse livers:

1. livers treated with Azathioprine 100mg/kg 5 days.
2. chlorambucil i.p. 15mg/kg 5 days this is the positive control as there is observed liver damage in these tissues seen by clinical pathology.
3. untreated livers.

All these tissues sampled at day 6.

Tissue prepared by adding 1 ml of lysis buffer (100mM potassium phosphate pH7.8,0.2% & 1% Triton X-100, 1mM DTT, 0.2mM PMSF and 5ug/ml leupeptin) to 100-150mg of ground tissue. Ribolyse in Hybaid Ribolyser for 2 x 10 second bursts on speed 6, microfuge for 5min 13000rpm. Remove supernatant from tube, aliquot and perform protein quantification assay. Estimation of protein content is done by the microtiter plate method for Bicinchoninic Acid Assay (BCA). BCA is used as Triton interferes with other protein assays such as Bradford Protein assay.

5.3 Assay (I)

Protein mix of interest is labelled with a fluorescent dye (e.g. molecular probes Alexa 488 protein labeling kit). The reaction is stopped using hydroxylamine and excess dye is removed by column separation. The fluorescently labelled protein is then added to PRU's attached to a surface. The position of binding and thus identity of the protein can then be identified. The protein mixture can be "spiked" with a protein which is an antigen for a PRU on the grid this then acts as a positive control to ensure binding has occurred.

Preparation of protein mixture.

Make protein mixes, typically a lysate with a positive control "spike" e.g. a protein which is the antigen to an antibody spotted on the grid. To label the protein mix with most molecular probes dyes the total concentration of the mix needs to be ~2mg/ml. Label protein mix and controls (spikes) with dyes (Molecular probes) using AlexaTM Protein Labeling Kit Protocol or FluoReporter BODIPY FL Protein Labeling Kit - this protocol was adapted for BODIPY 630/650 and BODIPY 650/665 as follows:

Add 20ul of Dye to 200ul lysate conc~1mg.
Leave in the dark for 1 hour gently inverting occasionally. Add 7ul of prepared hydroxylamine, pH 8.5 and leave for 30 min. Place in a spin column, containing 30,000 MW size exclusion resin in PBS (BIORAD) for 3mins 11000g. If any precipitate spin for 5 minutes and only take supernatant.

Binding of proteins to a PRU Array

All buffers added 20mls per slide.
Wash slides with 0.05% Tween /PBS buffer for 5 minutes
To saturate non specific protein binding sites block with Blocking buffer for 1 hour Wash in 0.05% Tween /PBS buffer
SCAN with Scanner Array (Molecular Dynamics) - keep slides from drying out these are used as a background control.
Due to drying out separate slides have to be made as controls for grids prepared on PVDF or nitro-cellulose.
Add 100ul dye + protein mixture to slides and leave in dark for 1 hour, keep in the dark as much as possible from this point.
Wash 2x for 5mins in TBST Wash Buffer
Wash 1x TBS Wash Buffer remove Tween
Add a couple of drops of Vectashield (Vector laboratories) to nitrocellulose slides (Grace Biolabs) this is to get a refractive index close to that of glass.
Place membrane on a glass slide and cover with a coverslip if using PVDF membrane. SCAN with Scanner Array (MD)

Buffers:

Carbonate Buffer pH 9.5, 0.2M per litre:

| | | |
|---|---|---|
| NaHCO$_3$ (Sigma) | 10.5g | |
| Na$_2$CO$_3$ (Sigma) | 5.16g | 1L. with distilled water. |

Or Carbonate-Bicarbonate buffer capsules 0.05M buffer, pH9.6. (Sigma)

TBS Wash Buffer pH 8.0 per litre:

| | | |
|---|---|---|
| 0.5M Tris-HCL (Sigma) | 20ml | pH8 |
| 0.15M NaCl (Sigma) | 8.8g | 1L. with distilled water. |

Dissolve the components in 800ml of deionized water. Adjust to pH 8.0 and bring final volume to 1L.

TBST Wash Buffer pH 8.0 per litre:

| | | |
|---|---|---|
| 0.5M Tris-HCL (Sigma) | 20ml | pH8 |
| 0.15M NaCl (Sigma) | 8.8g | |
| 0.05% Tween-20 (Sigma) | 1L. with distilled water. | |

Blocking buffer - make fresh:

1x PBS (Sigma)

0.2%(w/v) Marvel - dried skimmed milk powder

0.1%(w/v) Tween 20 (Sigma)

Lysis buffer: - This buffer does not contain Tris (Tris contains a primary amine and most of the molecular probe dyes react with any primary amine).
100mM potassium phosphate pH7.8 (Sigma)
0.2% Triton X-1008 (Sigma)
1mM DTT (Sigma) add just before use
0.2mM PMSF (Sigma) made up in isopropanol (Sigma) add just before use
5ug/ml leupeptin (Sigma) add just before use.

The slides are scanned at this first stage to determine background fluorescence of the surface (silane/ glutaraldehyde, nitro-cellulose or PVDF) and of the protein recognition units. Background fluorescence can be limited by optimising the concentration of the protein recognition units and also by choosing a dye which is in a range where there is least fluorescence of the protein recognition units.

Dyes were chosen by considering their level of binding to a protein mix and looking at their * Absorption (Abs) and fluorescence emission (Em) maxima. Spectra are detailed in Molecular Probes literature

Dyes are chosen to give minimal background fluorescence and with an emmission spectra suitable for the chosen apparatus of detection.

An Array Scanner- Generation III (Molecular Dynamics) was used:

| 532_multiples | excitation green 532nm | R_1 | 575B emission |
| | | R_2 | 675B " |
| | | R_3 | 620L " |
| | | R_4 | 720L " |
| 633_multiples: | excitation red 633nm | as above | |

Also a Storm (Molecular Dynamics) can be used for membranes only.

| Dye | Abs* (nm) | Em * (nm) |
| --- | --- | --- |
| Alexa 488 | 491 | 515 |
| Alexa 532 | 523 | 548 |
| Alexa 546 | 553 | 569 |
| Alexa 568 | 573 | 596 |
| Alexa 594 | 585 | 610 |
| | | |
| Bodipy 630/650 | 630 | 650 |
| Bodipy 650/665 | 650 | 670 |

Results

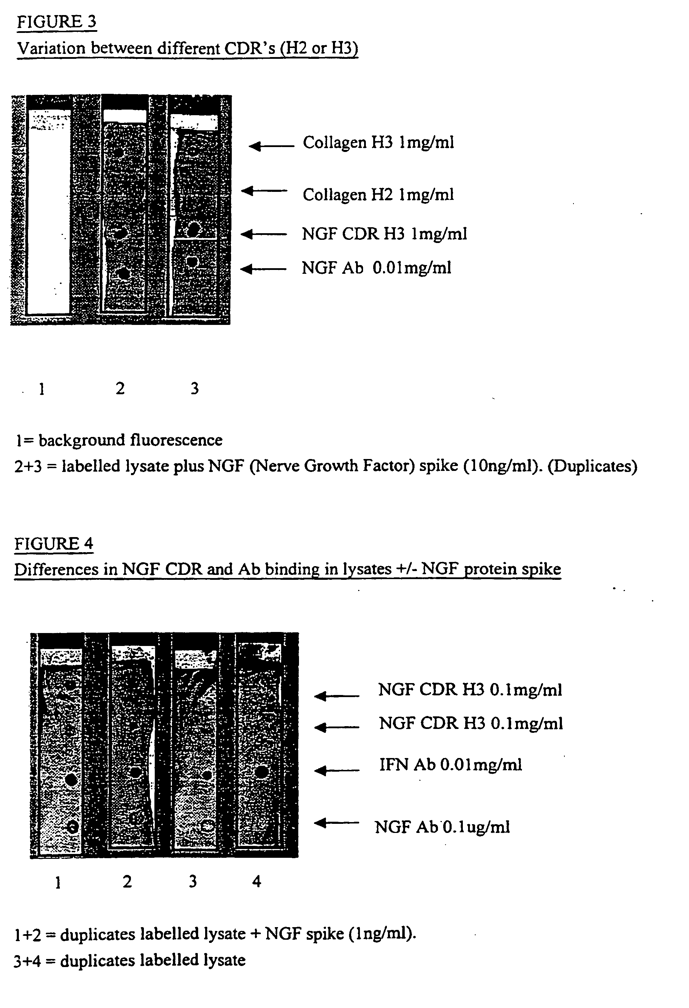

Figure 3 show the Variation between different CDR's (H2 or H3).
Collagen CDR H3 binds to collagen in the lysate and can be detected. Collagen CDR H2 shows no binding and NGF CDR H3 binds as strongly to the spiked lysate as NGF antibody (0.01mg/ml).
Figure 4 shows Differences in NGF CDR and Ab binding in lysates +/- NGF protein spike.
This shows binding by NGF CDR's and Ab in lysates with and without a NGF protein spike.
This experiment demonstrates the use of PRU arrays in detecting proteins in a mixture. The array can be built up with more PRU's relevant to the tissue or treatment of interest, in this case the assay is performed using lysates prepared from treated and untreated animals (e.g. mice treated with Azathioprine and control mice) to determine protein changes that occur when the animal is subjected to a treatment.

5.4 Assay (II)

Biotin/avidin method: A method of noncovalent conjugation is to make use of the natural strong binding of avidin for the small biotin molecule. Each avidin molecule contains a maximum of four biotin-binding sites, the interaction can be used to enhance the signal strength in the system of Assay (II).
Roche biotin labeling kit contains modification reagent that can add a functional biotin group to proteins. Biotin-labelled reagents are normally coupled to proteins through free amino groups. Biotin-labelled reagents are detected and quantitated by using avidin or streptavidin. These proteins bind tightly to biotin forming an essentially irreversible complex

($K_a$ 1014 mol$^{-1}$). NeutrAvidin biotin-binding protein is a protein that has been processed to remove the carbohydrate and lower its isoelectric point- this can sometimes reduce background staining.

Method: Label the protein mix of interest with Biotin using Biotin protein labelling kit (Roche) and pass though a column to remove excess biotin. Bind the protein-mix-biotin to the PRU slide for 1 hour at room temperature (RT). Wash off excess. Wash slide with Streptavidin-dye e.g. (NeutrAvidin TM- Alexa 488 conjugate (Molecular Probes) or Streptavidin, Alexa 546 conjugate (Molecular Probes)) for 1 hour RT. Wash and SCAN.

**Claims**

1. A method of predicting the toxic effect of a selected test compound on a mammalian subject, said method comprising the steps of;

   (a) providing a plurality of identical spatial arrays of protein recognition units;
   (b) exposing a spatial array of step (a) to a protein or peptide containing mixture extracted and isolated from a target cell, tissue or biological organism that has been exposed to a known toxicant and detecting the binding of proteins or peptides to the protein recognition units;
   (c) exposing a spatial anay of step (a) to a protein or peptide containg mixture extracted and isolated from a target cell, tissue or biological organism that has not been exposed to the known toxicant of step (b) and detecting the binding of proteins or peptides to the protein recognition units.
   (d) comparing the binding pattern of step (b) and step (c) to identify any difference in binding between the target exposed to the known toxicant and the non-exposed target, said difference being indicative of an effect on protein expression in the target exposed to said known toxicant;
   (e) repeating steps (b), and (d) optionally step (c) with another known and structurally distinct toxicant having a common toxic effect on mammalian subjects as the known toxicant of step (b) so as to generate a "fingerprint" binding pattern characteristic of said common toxic effect;
   (f) repeating steps (b) and (d), optionally steps (c) with said selected test compound to generate a test binding pattern wherein substantial identity between the fingerprint of step (e) and the test binding pattern indicates that said test compund shares said common toxic effect.

2. The method according to claim 1 wherein the spatial array comprises protein recognition units immobilised on a solid or semi-solid support.

3. The method according to claim 1 or 2 wherein the protein recognition units are selected from minimal recognition units, antibodies or antibody fragments, high affinity peptides and complementarity determining regions.

4. The method according to claim 3 wherein the protein recognition units are complementarity determining regions.

5. A method as claimed in any one of the preceding claims wherein the detection of binding is achieved using a staining method or using radiolabelling or chemical labelling, the labelling being performed either before or after the binding step.

6. A method according to any one of the preceding claims wherein detection of binding comprises scanning the spatial array using an optical or laser scanner and determining the co-ordinates of binding activity by a computing means.

7. The method according to any one of the preceding claims wherein the protein or peptide containing mixture is a cell extract isolated from a biological organism.

**Patentansprüche**

1. Verfahren zur Vorhersage der toxischen Wirkung einer ausgewählten Testverbindung auf ein Säugetier, wobei das Verfahren die folgenden Schritte umfaßt:

   (a) Bereitstellen einer Mehrzahl identischer räumlicher Felder von Proteinerkennungseinheiten;
   (b) Inkontaktbringen eines räumlichen Feldes aus Schritt (a) mit einer protein- oder peptidhaltigen Mischung, die aus einer Zielzelle, einem Zielgewebe oder einem biologischen Zielorganismus extrahiert und isoliert wurde, die/das/der einem bekannten toxischen Stoff ausgesetzt wurde, und Detektieren der Bindung von Proteinen oder Peptiden an die Proteinerkennungseinheiten;

(c) Inkontaktbringen eines räumlichen Feldes aus Schritt (a) mit einer protein- oder peptidhaltigen Mischung, die aus einer Zielzelle, einem Zielgewebe oder einem biologischen Zielorganismus extrahiert und isoliert wurde, die/das/der nicht dem bekannten toxischen Stoff aus Schritt (b) ausgesetzt wurde, und Detektieren der Bindung von Proteinen oder Peptiden an die Proteinerkennungseinheiten;

(d) Vergleichen des Bindungsmusters aus Schritt (b) und Schritt (c) zur Identifizierung eines etwaigen Bindungsunterschieds zwischen dem Ziel, das dem bekannten toxischen Stoff ausgesetzt wurde, und dem nicht-ausgesetzten Ziel, wobei der Unterschied indikativ für eine Wirkung auf die Proteinexpression in dem Ziel ist, das dem bekannten toxischen Stoff ausgesetzt wurde;

(e) wiederholen der Schritte (b) und (d) und gegebenenfalls von Schritt (c) mit einem anderen bekannten und strukturell unterschiedlichen toxischen Stoff mit einer gemeinsamen toxischen Wirkung auf Säugetiere wie der bekannte toxische Stoff aus Schritt (b), um ein "Fingerabdruck"-Bindungsmuster zu erzeugen, das charakteristisch für die gemeinsame toxische Wirkung ist;

(f) Wiederholen der Schritte (b) und (d) und gegebenenfalls von Schritt (c) mit der ausgewählten Testverbindung, um ein Testbindungsmuster zu erzeugen, worin eine substantielle Identität zwischen dem Fingerabdruck aus Schritt (e) und dem Testbindungsmuster anzeigt, daß die Testverbindung die gemeinsame toxische Wirkung teilt.

2. Verfahren gemäß Anspruch 1, worin das räumliche Feld Proteinerkennungseinheiten umfaßt, die auf einem festen oder halbfesten Träger immobilisiert sind.

3. Verfahren gemäß Anspruch 1 oder 2, worin die Proteinerkennungseinheiten aus minimalen Erkennungseinheiten, Antikörpern oder Antikörperfragmenten, hochaffinen Peptiden und komplementaritätsbestimmenden Regionen ausgewählt sind.

4. Verfahren gemäß Anspruch 3, worin die Proteinerkennungseinheiten komplementaritätsbestimmende Regionen sind.

5. Verfahren gemäß einem der vorhergehenden Ansprüche, worin die Detektion der Bindung unter Verwendung eines Anfärbungsverfahrens oder unter Verwendung von Radiomarkierung oder chemischer Markierung erreicht wird, wobei die Markierung entweder vor oder nach dem Bindungsschritt durchgeführt wird.

6. Verfahren gemäß einem der vorhergehenden Ansprüche, worin die Detektion der Bindung das Durchmustern des räumlichen Feldes unter Verwendung eines optischen oder Laser-Scanners und das Bestimmen der Koordinaten der Bindungsaktivität mit einer Berechnungseinrichtung umfaßt.

7. Verfahren gemäß einem der vorhergehenden Ansprüche, worin die protein- oder peptidhaltige Mischung ein aus einem biologischen Organismus isolierter Zellextrakt ist.

**Revendications**

1. Procédé pour prédire l'effet toxique d'un composé de test sélectionné sur un sujet mammifère, ledit procédé comprenant les étapes consistant à :

(a) fournir une pluralité de réseaux spatiaux identiques d'unités de reconnaissance de protéine ;

(b) exposer un réseau spatial de l'étape (a) à un mélange contenant une protéine ou un peptide extrait et isolé d'une cellule, d'un tissu ou d'un organisme biologique cibles qui ont été exposés à une substance toxique connue et détecter la liaison des protéines ou des peptides aux unités de reconnaissance de protéine ;

(c) exposer un réseau spatial de l'étape (a) à un mélange contenant une protéine ou un peptide extrait et isolé d'une cellule, d'un tissu ou d'un organisme biologique cibles qui n'ont pas été exposés à la substance toxique connue de l'étape (b) et détecter la liaison des protéines ou des peptides aux unités de reconnaissance de protéine ;

(d) comparer le profil de liaison de l'étape (b) et de l'étape (c) pour identifier toute différence de liaison entre la cible exposée à la substance toxique connue et à la cible non exposée, ladite différence indiquant un effet sur l'expression protéique dans la cible exposée à ladite substance toxique connue ;

(e) répéter les étapes (b) et (d), facultativement l'étape (c), avec une autre substance toxique connue et de structure distincte ayant un effet toxique commun sur les sujets mammifères comme substance toxique connue de l'étape (b) afin de générer un profil de liaison « empreinte digitale » caractéristique dudit effet toxique commun ;

(f) répéter les étapes (b) et (d), facultativement l'étape (c), avec ledit composé de test sélectionné pour générer un profil de liaison de test dans lequel une identité substantielle entre l'empreinte digitale de l'étape (e) et le profil de liaison de test indique que ledit composé de test partage ledit effet toxique commun.

2. Procédé selon la revendication 1 dans lequel le réseau spatial comprend des unités de reconnaissance de protéine immobilisées sur un support solide ou semi-solide.

3. Procédé selon la revendication 1 ou 2 dans lequel les unités de reconnaissance de protéine sont choisies parmi des unités de reconnaissance minimale, des anticorps ou des fragments d'anticorps, des peptides d'affinité élevée et des régions de détermination de la complémentarité.

4. Procédé selon la revendication 3 dans lequel les unités de reconnaissance de protéine sont des régions de détermination de la complémentarité.

5. Procédé selon l'une quelconque des revendications précédentes dans lequel la détection de la liaison est réalisée en utilisant un procédé de coloration ou en utilisant un radiomarquage ou un marquage chimique, le marquage étant réalisé avant ou après l'étape de liaison.

6. Procédé selon l'une quelconque des revendications précédentes dans lequel la détection de la liaison comprend le balayage du réseau spatial en utilisant un scanner optique ou laser et la détermination des coordonnées de l'activité de liaison avec des moyens informatiques.

7. Procédé selon l'une quelconque des revendications précédentes dans lequel le mélange contenant la protéine ou le peptide est un extrait cellulaire isolé d'un organisme biologique.

# FIGURE 1

## Diagrammatic illustration of protein arrays for high throughput proteomics

Figure 1

proteins

grid with PRU's

allow binding

proteins are visualised

FIGURE 2

Diagrammatic representation of result from one compound at one concentration in Example 4

## FIGURE 3
## Variation between different CDR's (H2 or H3)

← Collagen H3 1mg/ml

← Collagen H2 1mg/ml

← NGF CDR H3 1mg/ml

← NGF Ab 0.01mg/ml

1   2   3

1= background fluorescence

2+3 = labelled lysate plus NGF (Nerve Growth Factor) spike (10ng/ml). (Duplicates)

## FIGURE 4
## Differences in NGF CDR and Ab binding in lysates +/- NGF protein spike

← NGF CDR H3 0.1mg/ml

← NGF CDR H3 0.1mg/ml

← IFN Ab 0.01mg/ml

← NGF Ab 0.1ug/ml

1  2  3  4

1+2 = duplicates labelled lysate + NGF spike (1ng/ml).

3+4 = duplicates labelled lysate